# EUROPEAN PATENT APPLICATION

(11) **EP 4 759 291 A1**
(43) Date of publication of application: **17.06.2026**
(21) Application number: 25220962.2
(22) Date of filing: 05.12.2025
(51) Int. Cl.: A61H 31/00

(54) **CHEST COMPRESSION SYSTEM RETAINER AND BACKBOARD ASSEMBLY FOR USE WITH A PATIENT TRANSPORT APPARATUS**

(30) Priority: 10.12.2024 US 202463730013 P
(71) Applicant: Stryker Corporation, Portage, MI 49002 (US)
(72) Inventor: HENEVELD Jr., William, Ross, Portage, MI 49024 (US)
(74) Representative: Röthinger, Rainer

(57) **Abstract**

A patient care system includes a backboard assembly configured to be coupled to a patient for concurrent movement therewith. The backboard assembly includes a backboard defining a longitudinal axis, and a patient harness coupled to the backboard for coupling the backboard to the patient. The patient care system also includes a chest compression system configured to provide automatic chest compressions to the patient. The chest compression system includes a driver frame including lateral driver mounts configured to be releasably coupled to the backboard at a plurality of frame mounting positions along the longitudinal axis to support a driver adjacent to the chest of the patient. The patient care system further includes a retainer for releasably coupling the backboard assembly to an intermediate frame of a patient transport apparatus in a coupled state to inhibit movement of the patient and the chest compression system relative to a patient support surface.

## Description

### CROSS-REFERENCE TO RELATED APPLICATION(S)

This patent application claims priority to and all the benefits of United States Provisional Patent Application No. 63/730,013 filed on December 10, 2024, the disclosure of which is hereby incorporated by reference in its entirety.

### BACKGROUND

Cardiopulmonary resuscitation (CPR) is a lifesaving technique useful in many medical emergencies in which a patient's breathing and/or heartbeat has stopped, such as for example following a heart attack or a near drowning. Chest compressions are a primary aspect of CPR and involve firmly compressing the chest of the patient to keep oxygenated blood flowing to the brain and other vital organs until more definitive medical treatment can restore a normal heart rhythm. The administration of CPR requires the effort and attention of a caregiver, such an emergency medical technician (EMT), who is consequently generally unable to perform other treatment modalities that may benefit the patient suffering the medical emergency. The caregiver may also need to put themselves in danger in order to administer CPR, such as during ambulatory transport of the patient.

Devices have been developed which provide automatic chest compressions. One such device is the LUCAS^{™} family of chest compression systems, available from Physio-Control, Inc. This type of chest compression system utilizes a mechanical plunger to provide the chest compressions with the appropriate force and at the appropriate intervals. One notably useful application of the chest compression system is during transport of a patient supported on a patient transport apparatus, such as hospital bed, a stretcher, a cot, and the like. Additionally, it will be appreciated that providing automatic chest compressions during ambulance transport-often associated with high-speed driving, risky maneuvers, and/or hazardous road conditions-may mitigate the need for caregivers to perform CPR while standing unrestrained in a confined space.

Due to the elevation of the patient support surface on which the patient is supported, especially during ambulatory transport, the caregivers or other treating medical professionals may need to closely monitor the stability of the chest compression system (and the patient) supported on the patient transport apparatus, and may need to provide attention to or otherwise manually assist with stabilizing and/or repositioning the chest compression system. As a result, the medical professionals may be inhibited from performing other types of treatment or patient cate. Moreover, in some circumstances, the caregiver may not be able to assist with stabilizing the chest compression system and may have to attend to other types of treatment or patient care. Furthermore, in some instances it may be desirable to continue providing chest compressions via the chest compression system once a patient has been moved off of the patient support surface.

Accordingly, there remains a need in the art to improve the retention of chest compression system relative to the patient and/or a patient transport apparatus.

### SUMMARY

One general aspect of the present disclosure includes a patient care system for treating a patient. The patient care system includes a patient transport apparatus including a base arranged for movement along floor surfaces, an intermediate frame arranged for movement relative to the base between a plurality of vertical configurations, and a patient support deck operatively attached to the intermediate frame and defining a patient support surface for supporting the patient. The patient care system also includes a backboard assembly configured to be coupled to the patient for concurrent movement with the patient. The backboard assembly includes a backboard defining a longitudinal axis, and a patient harness coupled to the backboard for coupling the backboard to the patient. The patient care system also includes a chest compression system configured to provide automatic chest compressions to the patient. The chest compression system includes a driver having a driver body movably supporting a plunger arranged for providing chest compressions to the patient, and a driver frame including lateral driver mounts configured to be releasably coupled to the backboard at a plurality of frame mounting positions along the longitudinal axis to support the driver adjacent to the chest of the patient. The patient care system further includes a retainer for releasably coupling the backboard assembly to the intermediate frame of the patient transport apparatus in a coupled state to inhibit movement of the patient and the chest compression system relative to the patient support surface.

Another general aspect of the present disclosure includes a patient care system for treating a patient. The patient care system includes a backboard assembly configured to be coupled to the patient for concurrent movement with the patient. The backboard assembly includes a backboard defining a longitudinal axis and including a plurality of backboard members configured for selective relative movement relative to each other, and a patient harness coupled to the backboard for coupling the backboard to the patient. The patient care system also includes a chest compression system configured to provide automatic chest compressions to the patient. The chest compression system includes a driver having a driver body movably supporting a plunger arranged for providing chest compressions to the patient, and a driver frame including lateral driver mounts configured to be releasably coupled to the backboard at a plurality of frame mounting positions along the longitudinal axis to support the driver adjacent to the chest of the patient.

### BRIEF DESCRIPTION OF THE DRAWINGS

Advantages of the present disclosure will be readily appreciated as the same becomes better understood by reference to the following detailed description when considered in connection with the accompanying drawings.
FIG. 1 is a perspective view of one configuration of a patient care system according to the present disclosure, including a patient transport apparatus, a backboard assembly, a chest compression system, and a retainer.
FIG. 2 is a perspective view of one configuration of a chest compression system according to the present disclosure.
FIG. 3 is a partial side perspective view of the patient care system illustrating the retainer coupled to an intermediate frame of the patient transport apparatus in a coupled state.
FIG. 4 is a partial side perspective view the patient care system illustrating the retainer decoupled from the patient transport apparatus in a decoupled state.
FIG. 5 is a side perspective view of the patient care system including the backboard assembly, chest compression system, and the retainer.
FIG. 6 is a perspective exploded view of the patient care system with the chest compression system spaced relative to one configuration of the backboard assembly including a plurality of mounting features.
FIG. 7A is a perspective view of the patient care system of FIG. 6 illustrating the chest compression system engaging a grouping of the mounting features of the backboard assembly such that the chest compression system is disposed at a rearward frame mounting position.
FIG. 7B is a perspective view of the patient care system of FIG. 6 illustrating the chest compression system engaging a different grouping of mounting features of the backboard assembly that the chest compression system is disposed at a forward frame mounting position.
FIG. 8 is a perspective exploded view of the patient care system with the chest compression system spaced relative to another configuration of the backboard assembly including a translator assembly.
FIG. 9A is a perspective view of the patient care system of FIG. 8 illustrating the chest compression system engaging the translator assembly of the backboard assembly such that the chest compression system is disposed at a rearward frame mounting position.
FIG. 9B is a perspective view of the patient care system of FIG. 8 illustrating the chest compression system engaging the translator assembly of the backboard assembly that the chest compression system is disposed at a forward frame mounting position.
FIG. 10A shows one configuration of the backboard including a plurality of backboard members in an assembled state.
FIG. 10B shows the backboard of FIG. 10A with the plurality of backboard members in a disassembled state.
FIG. 11A shows one configuration of the backboard including three backboard members hingedly connected and configured to pivot about respective hinge axes parallel to a longitudinal axis of the backboard.
FIG. 11B shows the backboard of FIG. 11A with the plurality of backboard members moving from a deployed state to a folded state.
FIG. 12A shows one configuration of the backboard including three backboard members hingedly connected and configured to pivot about respective hinge axes transverse to a longitudinal axis of the backboard.
FIG. 12B shows the backboard of FIG. 12A with the plurality of backboard members moving from a deployed state to a folded state.
FIG. 13A shows one configuration of the backboard including two backboard members hingedly connected and configured to pivot about a hinge axis parallel to a longitudinal axis of the backboard.
FIG. 13B shows the backboard of FIG. 13A with the plurality of backboard members moving from a deployed state to a folded state.
FIG. 14A shows one configuration of the backboard including two backboard members hingedly connected and configured to pivot about a hinge axis transverse to a longitudinal axis of the backboard.
FIG. 14B shows the backboard of FIG. 14A with the plurality of backboard members moving from a deployed state to a folded state.

### DETAILED DESCRIPTION

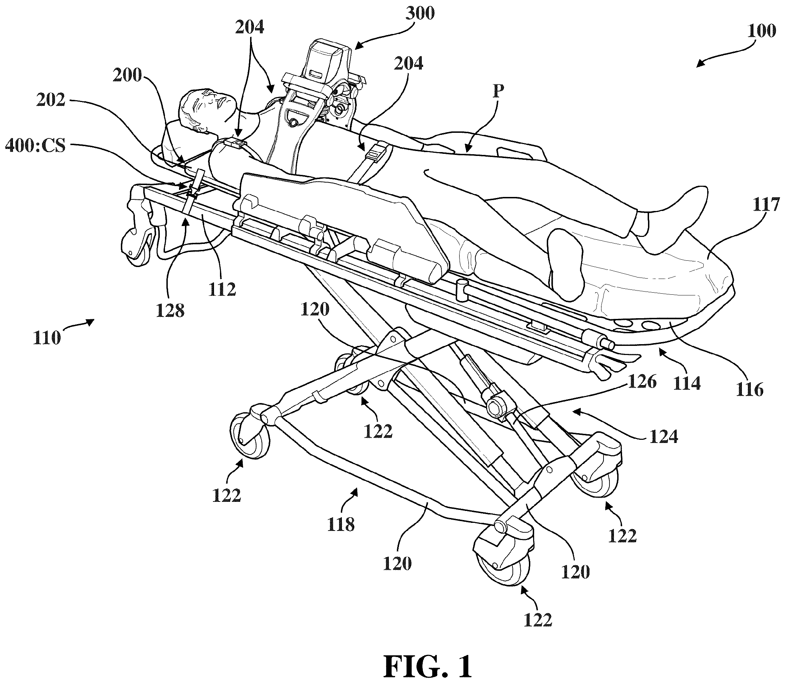

Referring to FIG. 1, a patient care system 100 is shown for treating a patient P in a health care and/or transportation setting. The patient care system 100 generally includes a patient transport apparatus 110 for supporting the patient P, a backboard assembly 200 configured to be coupled to the patient P for concurrent movement with the patient P, a chest compression system 300 configured to provide automatic chest compressions to the patient P, and a retainer 400 for releasably coupling the backboard assembly 200 to the patient transport apparatus 110. The patient transport apparatus 110 illustrated in FIG. 1 is realized as a cot. In other versions however, the patient transport apparatus 110 may be a hospital bed, stretcher, table, wheelchair, chair, or similar apparatus utilized in the transportation and care of a patient P.

As shown in FIG. 1, the patient transport apparatus 110 includes an intermediate frame 112 configured to support the patient P. The intermediate frame 112 may be operatively attached to a variety of components that aid in supporting and/or transporting the patient P. For example, in FIG. 1, the intermediate frame 112 is operatively attached to to a patient support deck 114 defining a patient support surface 116 for supporting the patient P. In some configurations, a mattress 117 may be disposed on the patient support surface 116 for supporting the patient P. The patient support deck 114 may be defined by one or more articulable deck sections, for example, a fowler deck section, a seat deck section, a leg deck section, and a head deck section, to facilitate care and/or transportation of the patient P in various patient positions. The intermediate frame 112 and corresponding deck sections can be like that shown in U.S. Patent Application Publication No. 2018/0303689 A1, which claims priority to U.S. Provisional Patent Application No. 62/488,441, filed on Apr. 21, 2017, entitled, "Emergency Cot With A Litter Height Adjustment Mechanism," the disclosures of which are hereby incorporated by reference in their entirety.

The patient transport apparatus 110 also includes a base 118 arranged for movement along floor surfaces. The base 118 may be generally rectangular and defined by a plurality of rails 120, but other configurations are contemplated. The base 118 may further include a plurality of caster wheel assemblies 122 operatively connected adjacent to each corner of the base 118 defined by the rails 120. As such, the patient transport apparatus 110 of FIG. 1 includes four caster wheel assemblies 122. The caster wheel assemblies 122 may be configured to swivel to facilitate turning of the patient transport apparatus 110. The caster wheel assemblies 122 may include a swivel locking mechanism to prevent the caster wheel assemblies 122 from swiveling when engaged. The caster wheel assemblies 122 may also include wheel brakes (not shown) to prevent rotation of the wheel.

The patient transport apparatus 110 may also include a lift mechanism 124 interposed between the base 118 and the intermediate frame 112. The lift mechanism 124 may be configured to move between a plurality of vertical configurations including an extended configuration where the intermediate frame 112 is elevated relative to the base 118, as shown in FIG. 1, and a retracted configuration (not shown) where the intermediate frame 112 is lowered such that it is in closer proximity to the base 118. The lift mechanism 124 can be like that shown in U.S. Patent Application Publication No. 2018/0303689 A1, incorporated above.

While moving between the plurality of vertical configurations, the lift mechanism 124 moves either the base 118 or the intermediate frame 112 relative to the other of the intermediate frame 112 or the base 118 depending on how the patient transport apparatus 110 is supported during use. For example, the patient transport apparatus 110 may be supported at the intermediate frame 112 when the patient transport apparatus 110 is being unloaded/loaded into an emergency response vehicle (not shown) and the patient transport apparatus 110 may be supported at the base 118 when the patient transport apparatus 110 is resting on a surface such as a hospital floor. In instances where the patient transport apparatus 110 is supported at the intermediate frame 112, the lift mechanism 124, while moving between the plurality of vertical configurations, moves the base 118 relative to the intermediate frame 112. In instances where the patient transport apparatus 110 is supported at the base 118, the lift mechanism 124, while moving between the plurality of vertical configurations, moves the intermediate frame 112 relative to the base 118.

The patient transport apparatus 110 may include a variety of components that allow the lift mechanism 124 to move between the plurality of vertical configurations. For example, the lift mechanism may be implemented as a scissor-type lift mechanism as illustrated in FIG. 1, but other configurations including but not limited to column-type lift mechanisms are contemplated. In one example, the patient transport apparatus 110 may include a mechanism like that shown in U.S. Patent Application Publication No. 2018/0303689 A1, incorporated above.

Those having ordinary skill in the art will appreciate that the lift mechanism 124 may move between the plurality of vertical configurations due to a patient care provider applying a manual action to the lift mechanism 124, or components thereof. Additionally, or alternatively, the patient transport apparatus 110 may include one or more actuators 126, which may be coupled to any suitable component of the lift mechanism 124 and may be configured to move the lift mechanism 124 between the plurality of vertical configurations. In some versions, the actuator 126 is realized as a hydraulic linear actuator. However, in other versions, the actuator 126 may not be a hydraulic linear actuator. The actuator 126 may be any actuator suitable for actuating the lift mechanism 124 such that the lift mechanism 124 moves between the plurality of vertical configurations. For example, the actuator 126 may be an electric motor, a servo motor, a pneumatic actuator, or any other suitable actuator. One example of an actuator 126 is further described in U.S. Pat. No. 7,398,571, filed on Jun. 30, 2005, entitled, "Ambulance Cot and Hydraulic Elevating Mechanism Therefor," the disclosure of which is hereby incorporated by reference in its entirety. Furthermore, techniques for utilizing actuator 126 to manipulate the components of the patient transport apparatus 110 can be like those described in U.S. Patent Application Publication No. 2018/0303689 A1, incorporated above. Other configurations are contemplated.

As noted above, the patient care system 100 includes the backboard assembly 200 configured to be coupled to the patient P for concurrent movement with the patient P. The backboard assembly 200 includes a backboard 202 defining a longitudinal axis LA. The backboard 202 may be comprised of materials such as metal, plastic, the like, or a combination thereof such that the backboard 202 has sufficient structural integrity/rigidity to at least partially support the patient P and the chest compression system 300. Exemplary configurations of the backboard 202 are described in further detail below. The backboard assembly 200 further includes a patient harness 204 coupled to the backboard 202 for coupling the backboard 202 to the patient P. Accordingly, where coupled to the patient P, the backboard 202 is configured to move concurrently with the patient P, as described in further detail below.

As depicted in the drawings, the patient harness 204 may include a plurality of straps 206 configured to engage the patient P to couple the backboard 202 to the patient P. In order for the patient harness 204 to secure the patient P to the backboard 202 with adequate strength to withstand the forces of transport or vehicular collision, the patient harness 204 may be at least partially formed of a suitably strong material. For example, the plurality of straps 206 may be formed of strong fabric or webbing, such as polypropylene, polyester, nylon, analogous materials, combinations thereof, and the like, which may be coated, treated, or otherwise configured in various ways. Other configurations are contemplated.

In some versions, the plurality of straps 206 includes a pair of shoulder straps 208A, 208B configured to engage the shoulders of the patient P to couple the backboard 202 to the patient P. The plurality of straps 206 may additionally or alternatively include torso straps 210 configured to engage the torso of the patient P to couple the backboard 202 to the patient P. It is also contemplated that the plurality of straps 206 may additionally or alternatively include leg straps, hip straps, waist straps, neck straps, etc. configured to engage the patient P to couple the backboard 202 to the patient P. In the illustrated configurations, each of the pair of shoulder straps 208A, 208B and the torso straps 210 include corresponding strap portions that attach via respective buckles 212 to couple the backboard 202 to the patient P. It will be appreciated that patient harness 204 may include different configurations and/or arrangements of straps, buckles, and the like. Although not explicitly shown in the drawings, it will be appreciated that lengths of the plurality of straps 206 of the patient harness 204 may be adjustable by any suitable length-adjustment apparatus.

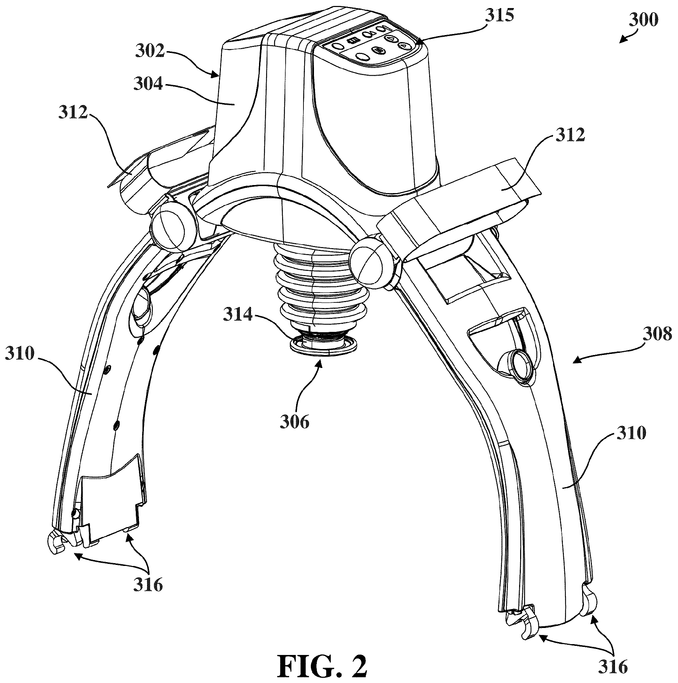

As noted above, the patient care system 100 includes the chest compression system 300 for providing automatic chest compressions to the patient P. As best shown in FIG. 2, the chest compression system 300 generally includes a driver 302 having a driver body 304 movably supporting a plunger 306 arranged for providing chest compressions to the patient P, and a driver frame 308 for supporting the driver 302 adjacent to a chest of the patient P. In order to support the driver 302 relative to the chest of the patient P, the driver frame 308 includes lateral driver mounts 310 operatively attached to the driver body 304. The chest compression system 300 may also include handles 312 coupled to the driver frame 308, such as to lateral driver mounts 310, at a suitable position for securing the upper extremities of the patient P to, among other things, avoid interference with the operation of the chest compression system 300.

It will be appreciated that the driver body 304 and the lateral driver mounts 310 may be formed from separate components that are coupled together or may be formed integrally in some versions. The driver body 304 houses a number of the electromechanical components of the chest compression system 300, including a piston rod 314 which extends to the plunger 306 as shown in FIG. 2. The piston rod 314 is powered by a motor (not shown) which moves the piston rod 314, and therefore the plunger 306, between retracted positions and extended positions. The plunger 306 may also be actuated with any suitable form of propulsion, for example, electric, electromagnetic, pneumatic, and the like. As the plunger 306 moves between positions while situated on the patient P, the patient P receives automatic chest compressions analogous to those which would otherwise be provided by a physician performing CPR.

In order to allow the user to control the chest compression system 300, a control panel 315 may be disposed on the driver body 304. The control panel 315 is configured to receive inputs from the user, which may have or facilitate carrying out various functions. For example, start, stop, reset, and similar functions may be used as inputs sent to the chest compression system 300 via the control panel 315. As shown in FIG. 2, the control panel 315 may include depressible buttons to provide these types of inputs. In other versions, the control panel 315 may be remote from the chest compression system 300. For example, the control panel 315 may take the form of a transceiver located anywhere on the chest compression system 300 which receives control signals from a remote source, such as a controller, smartphone, tablet, keyboard, and the like.

Certain operative and structural features of the chest compression system 300 are further disclosed in U.S. Pat. No. 7,226,427, issued Jul. 5, 2007, and entitled SYSTEMS AND PROCEDURES FOR TREATING CARDIAC ARREST, the entire contents of which are hereby incorporated by reference. Additionally, other features of the chest compression system 300 are disclosed in U.S. Patent Application Publication No. 2019/0117502, published Apr. 25, 2019, and entitled PATIENT SUPPORT APPARATUS FOR RELEASABLY SECURING A CHEST COMPRESSION SYSTEM, the entire contents of which are hereby incorporated by reference.

As described in further detail below, the lateral driver mounts 310 of the chest compression system 300 are configured to be releasably coupled to the backboard 202 at a plurality of frame mounting positions MP along the longitudinal axis LA to support the driver 302 adjacent to the chest of the patient P. Here, the lateral driver mounts 310 are of a suitable length to at least partially define a patient volume of sufficient size to receive the torso of the patient P. In these configurations, when the patient P is disposed on the patient support surface 116 of the patient transport apparatus, the backboard 202 is arranged between the patient P and the patient support surface 116. As the driver 302 is providing downward force via the plunger 306, the backboard 202 may provide a corresponding/reciprocal upward force. This ensures that the downward force provided by the chest compression system 300 is absorbed by the chest of the patient P and is not instead dissipated to, for example, the patient support surface 116.

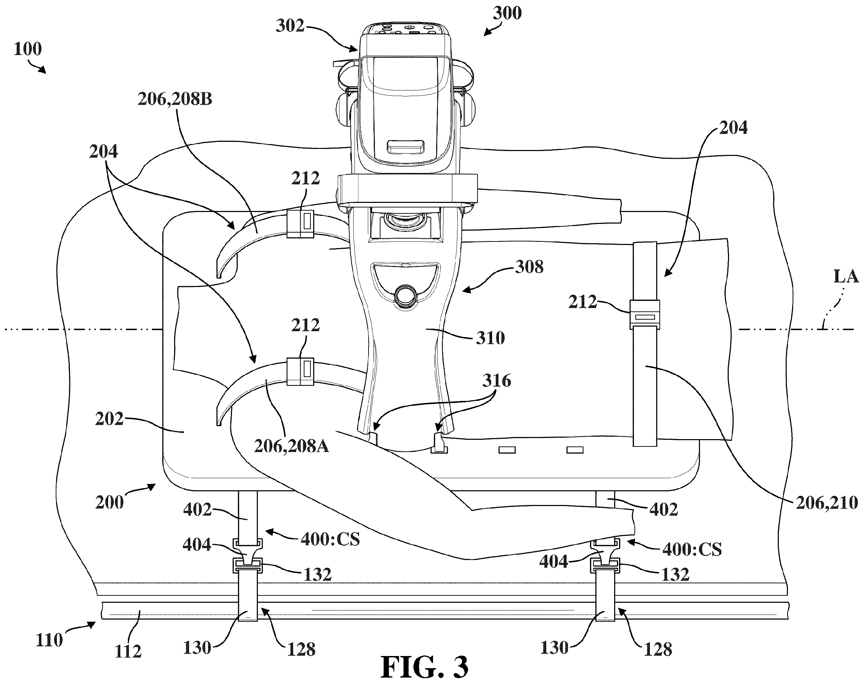

Referring to FIG. 1 and FIGS. 3-4, as noted above, the patient care system 100 includes the retainer 400 for releasably coupling the backboard assembly 200 to the patient transport apparatus 110. More specifically, as best shown in FIG. 3, the retainer 400 is configured to releasably couple the backboard assembly 200 to the intermediate frame 112 of the patient transport apparatus 110 in a coupled state CS to inhibit movement of the patient P and the chest compression system 300 relative to the patient support surface 116. Accordingly, the patient P and the chest compression system 300 may be inhibited from moving relative to the patient support surface 116 by virtue of the retainer 400 coupling the backboard assembly 200 to the intermediate frame 112 of the patient transport apparatus 110 in the coupled state CS such that the patient harness 204 is not directly coupled to the intermediate frame 112 in the coupled state CS. By aligning the patient P relative to the backboard 202 via the patient harness 204, as opposed to aligning the patient P to the intermediate frame 112, alignment of the chest compression system 300 with the patient P is improved.

As best shown in FIG. 3, the retainer 400 may include a plurality of tethers 402 extending between the backboard 202 and the intermediate frame 112. In order for the retainer 400 to secure the backboard assembly 200 to the patient transport apparatus 110 with adequate strength to withstand the forces of transport or vehicular collision, the retainer 400 may be at least partially formed of a suitably strong material. For example, the plurality of tethers 402 may be formed of strong fabric or webbing, such as polypropylene, polyester, nylon, analogous materials, combinations thereof, and the like, which may be coated, treated, or otherwise configured in various ways. Other configurations are contemplated.

In some configurations, the tethers 402 may extend both longitudinally and laterally relative to the backboard assembly 200, which helps ensure that the backboard assembly 200 (and, thus, the patient P and chest compression system 300) is retained in a stable arrangement and is inhibited from moving relative to the patient P along the longitudinal axis LA and/or laterally relative to longitudinal axis LA. It will be appreciated that limiting movement of the backboard assembly 200 (and, thus, the patient P and chest compression system 300) relative to the patient transport apparatus 110 can promote improved usability and safety for caregivers, patients, and others during various use case scenarios (e.g., during transportation in an ambulance). Additionally, although not explicitly shown in the drawings, it will be appreciated that lengths of the plurality of tethers 402 may be adjustable by any suitable length-adjustment apparatus.

As best shown in FIGS. 1 and 3, the intermediate frame 112 of the patient transport apparatus 110 may define a plurality of mounting points 128 configured to engage a respective tether 402 of the plurality of tethers 402 to releasably couple the backboard assembly 200 to the intermediate frame 112 in the coupled state CS. In the illustrated version, the plurality of mounting points 128 each include a mounting strap 130 coupled to the intermediate frame 112 of the patient transport apparatus 110. In some versions, the mounting straps 130 may be realized as "loops" of webbing which can be wrapped around, passed through, or otherwise secured with portions of the intermediate frame 112 or other parts of the patient transport apparatus 110. The mounting strap 130 may be fixed relative to the intermediate frame 112 or configured to move longitudinally along the intermediate frame 112 for adjustability. Other configurations are contemplated.

The plurality of mounting points 128 may each additionally include a mounting ring 132 supported by a respective mounting strap 130 and configured to engage a respective tether 402 of the plurality of tethers 402 to releasably couple the backboard assembly 200 to the intermediate frame 112 in the coupled state CS. For example, each of the plurality of tethers 402 may include a clip 404 (e.g., a carabiner or the like) configured to engage the mounting ring 132 to couple the plurality of tethers 402 to respective mounting strap 130 in the coupled state CS. Other configurations for providing the plurality of mounting points 128 contemplated. For example, in some configurations a mounting ring 132 may be coupled to or formed integrally with the intermediate frame 112. In another example, instead of a mounting ring 132, the mounting straps 130 may each include a buckle configured to engage a corresponding buckle of the plurality of tethers 402. It will be appreciated that releasable coupling via the retainer 400 between the intermediate frame 112 and the backboard assembly 200 may be realized in a number of different ways, and configurations other than those illustrated throughout the drawings are contemplated by the present disclosure.

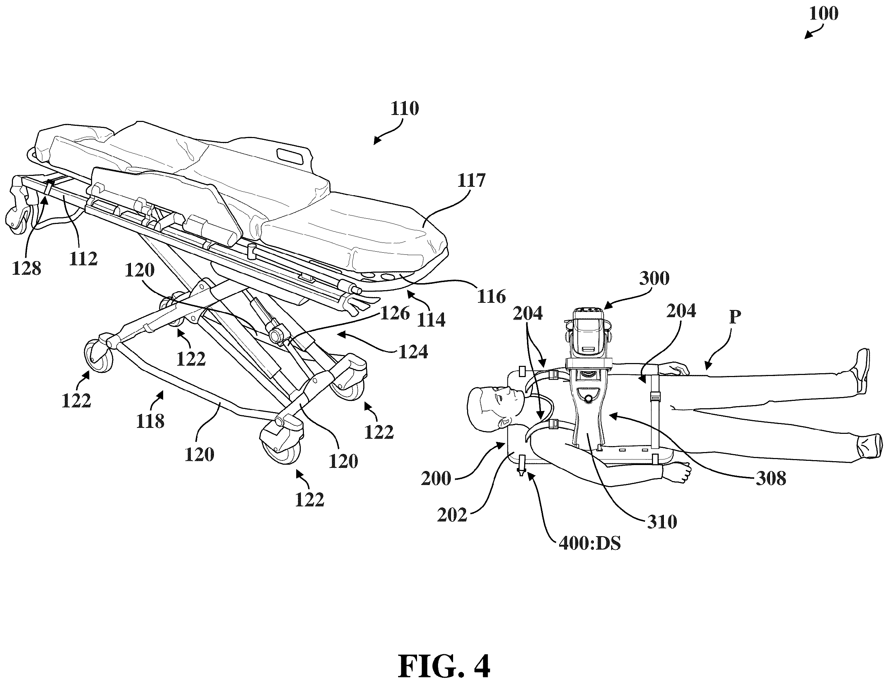

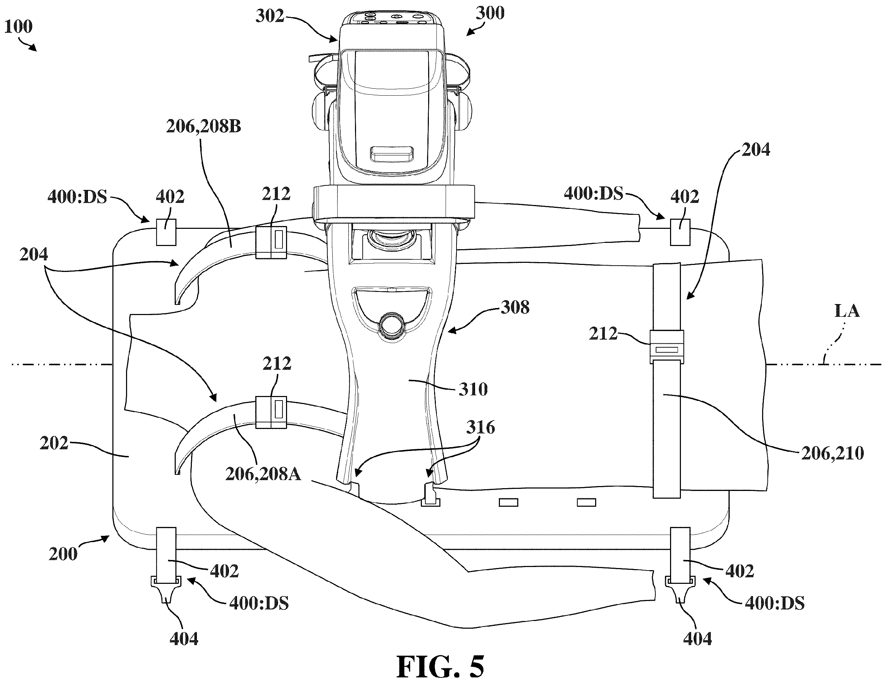

The retainer 400 is operable between the coupled state CS (best shown in FIGS. 1 and 3) and a decoupled state DS (best shown in FIGS. 4 and 5). In the decoupled state DS, the backboard assembly 200 is decoupled from the intermediate frame 112 of the patient transport apparatus 110 for permitting concurrent movement of the patient P, the backboard assembly 200, and the chest compression system 300 relative to the patient support surface 116. Accordingly, as illustrated in FIGS. 4 and 5, in the decoupled state DS, the backboard assembly 200 remains coupled to the patient P for concurrent movement with the patient P to allow the driver of the chest compression system 300 to continue providing chest compressions to the patient P as the retainer 400 moves from the coupled state CS to the decoupled state DS. For example, where the retainer 400 is in the decoupled state DS, the backboard assembly 200 (and, thus, the patient P and chest compression system 300) is released from being coupled to the patient transport apparatus 110 such that the patient P may be moved from the patient transport apparatus 110 by caregivers while the chest compression system 300 continues provide chest compressions to the patient P.

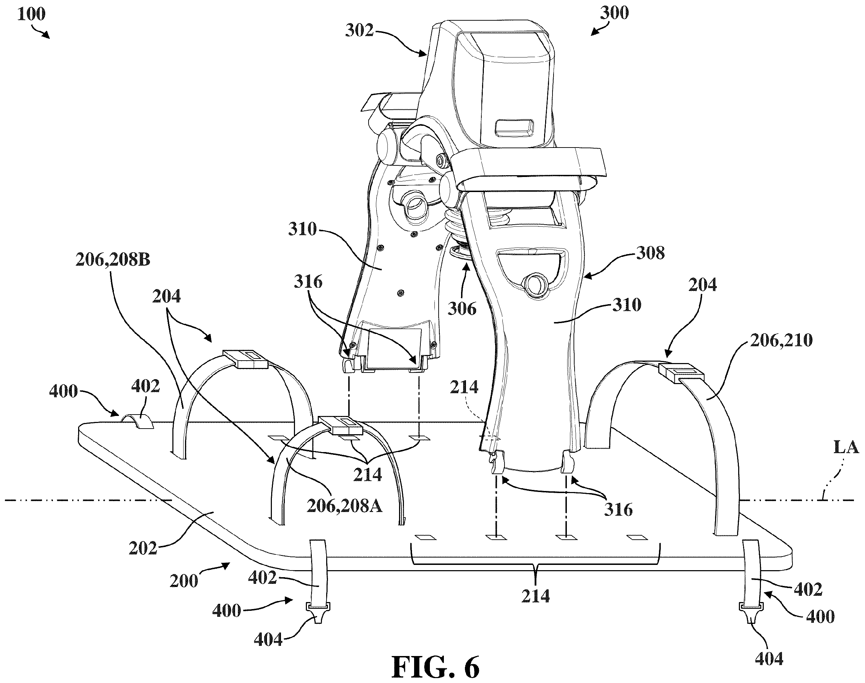

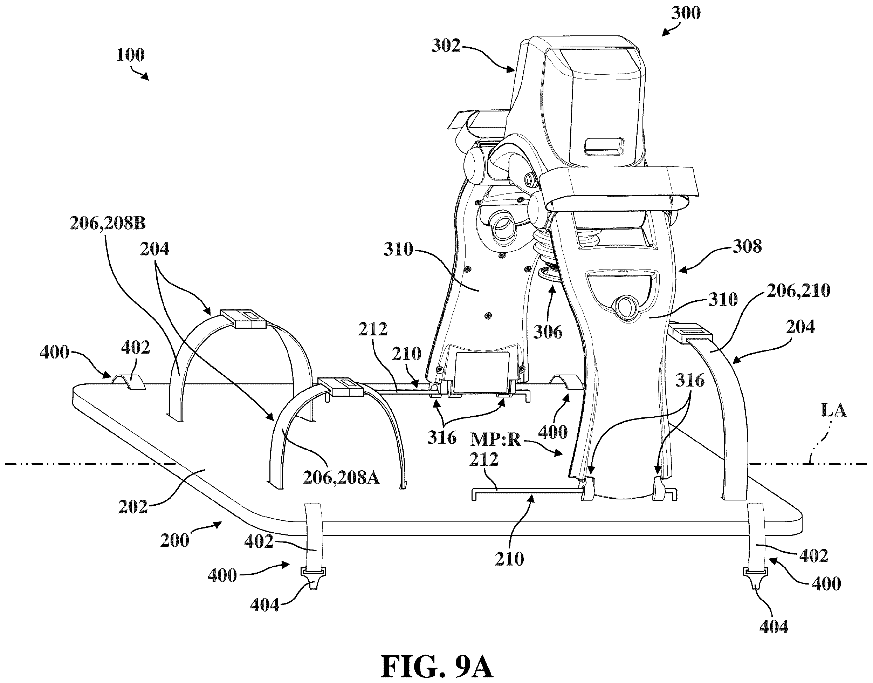

As noted above, the lateral driver mounts 310 of the chest compression system 300 are configured to be releasably coupled to the backboard 202 at a plurality of frame mounting positions MP along the longitudinal axis LA to support the driver 302 adjacent to the chest of the patient P. As described in further detail in the context of FIGS. 6-9B, a variety of configurations of the backboard 202 to define the plurality of frame mounting positions MP along the longitudinal axis LA are contemplated. In any event, at a junction between each of the lateral driver mounts 310 and the backboard 202, a locking mechanism 316 may be provided to releasably couple an end of the lateral driver mounts 310 to the backboard 202 at one of the plurality of frame mounting positions MP along the longitudinal axis LA. Consequently, the chest compression system 300 is configured to be separable from the backboard 202 for various reasons, such as storage, transport, and disengaging the chest compression system 300 from the patient P. In some examples, the backboard 202 defines a length L along the longitudinal axis LA and defines a width W along a lateral axis transverse to the longitudinal axis LA, wherein the length L is greater than the width W. In examples where the length L is greater than the width W, the backboard 202 provides more adjustability along the longitudinal axis LA between the plurality of frame mounting positions MP.

Here, it will be appreciated that separability of the chest compression system 300 from the backboard 202 facilitates quick positioning and engagement of the chest compressions system 300 with the patient P. For example, during use, the backboard 202 may be situated on the patient support surface 116, and the patient P may be positioned on top of the backboard 202. After the patient P has been positioned on the backboard 202, the chest compressions system 300 (particularly, the lateral driver mounts 310), is positioned near opposing ends of the backboard 202, and the locking mechanisms 316 may then be engaged with the backboard 202 at one of the plurality of frame mounting positions MP along the longitudinal axis LA to couple the lateral driver mounts 310 (and, thus, the chest compression system 300) to the backboard assembly 200. One or both of the locking mechanisms 316 may be disengaged to facilitate adjustment of the chest compression system 300 relative to the patient P (e.g. to adjust between the plurality of frame mounting positions MP along the longitudinal axis LA) and/or to facilitate removal of the chest compression system 300 after use. For example, the locking mechanisms 316 may include a releasing member arranged to receive an input from a user to disengage the lateral driver mounts 310 from the backboard 202.

As noted above, the chest compression system 300 is configured to be releasably coupled to the backboard 202 at a plurality of frame mounting positions MP along the longitudinal axis LA. Stated differently, the position of the chest compression system 300 along the longitudinal axis LA may be adjustable to enable a caretaker to properly align the chest compression system 300 with the chest of the patient P. In one example, referring to FIGS. 6-7B, the plurality of frame mounting positions MP may be a discrete plurality of frame mounting positions MP. In these examples, the backboard 202 may define a plurality of mounting features 214 defining the discrete plurality of frame mounting positions MP and configured to be releasably coupled with the lateral driver mounts 310. For example, the plurality of mounting features 214 of the backboard 202 may be realized as a plurality of voids, members, etc. which are configured to receive or otherwise engage the locking mechanisms 316 to releasably couple the lateral driver mounts 310 to the backboard 202.

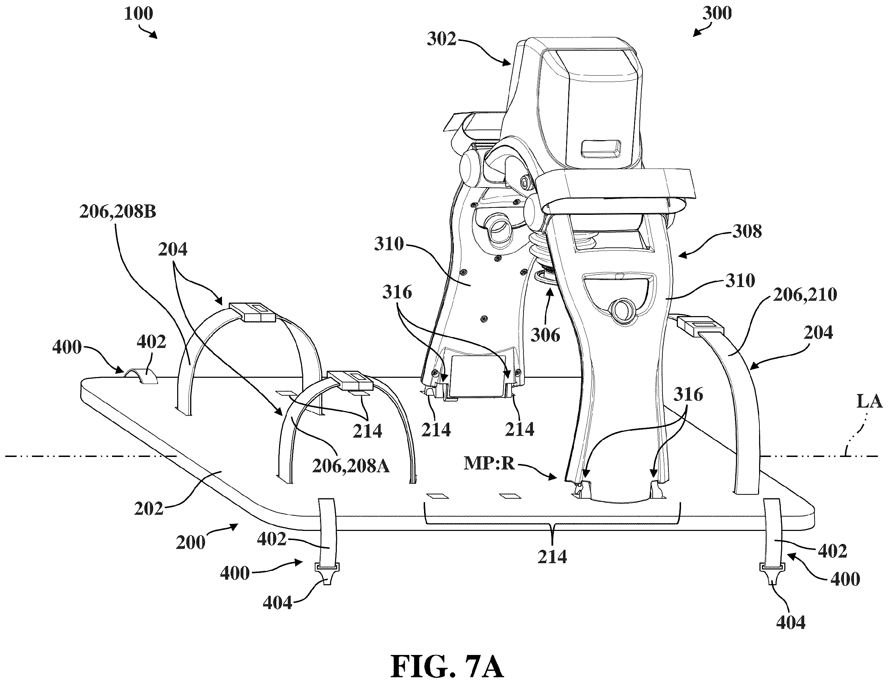

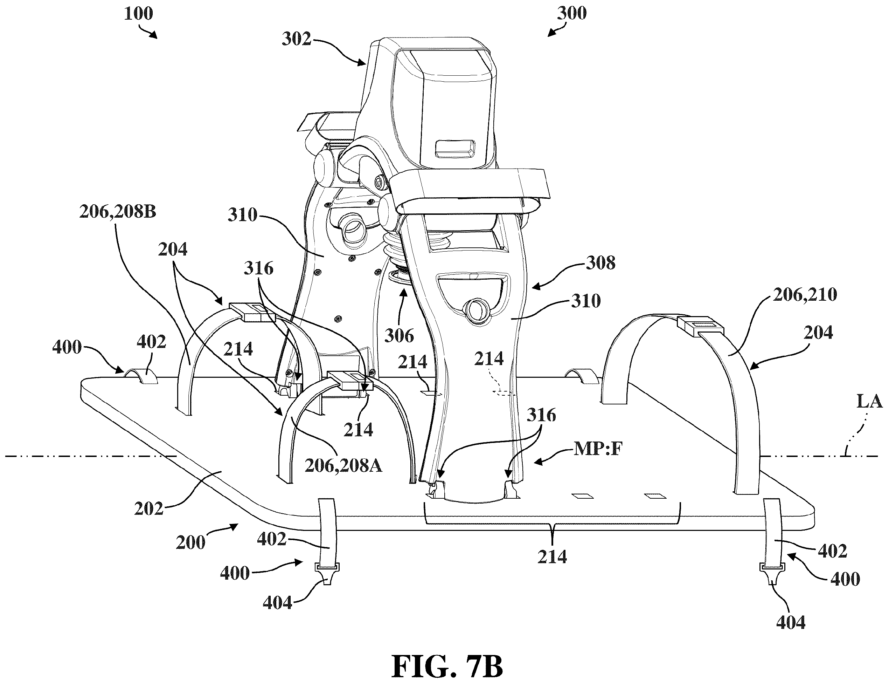

As illustrated in the configuration of FIGS. 6-7B, the plurality of mounting features 214 may be spaced along the longitudinal axis LA of the backboard 202 to define the discrete plurality of frame mounting positions MP. For example, Figure 7A shows the locking mechanisms 316 of the chest compression system 300 engaging a grouping of the plurality of mounting features 214 of the backboard 202 such that the chest compression system 300 is disposed at a rearward frame mounting position MP:R. Figure 7B shows the locking mechanisms 316 of the chest compression system 300 engaging a different grouping of the plurality of mounting features 214 such that the chest compression system 300 is disposed at a forward frame mounting position MP:F. Although not illustrated, it should be appreciated that the locking mechanisms 316 of the chest compression system 300 may engage intermediate groupings of the plurality of mounting features 214 of the backboard 202 such that the chest compression system 300 is disposed at one or more intermediate frame mounting positions between the forward frame mounting positions MP:F and the rearward frame mounting positions MP:R It is contemplated that the plurality of mounting features 214 may include any suitable number of mounting features 214 spaced along the longitudinal axis LA to define the discrete plurality of frame mounting positions MP. Other configurations of defining the discrete plurality of frame mounting positions MP are contemplated.

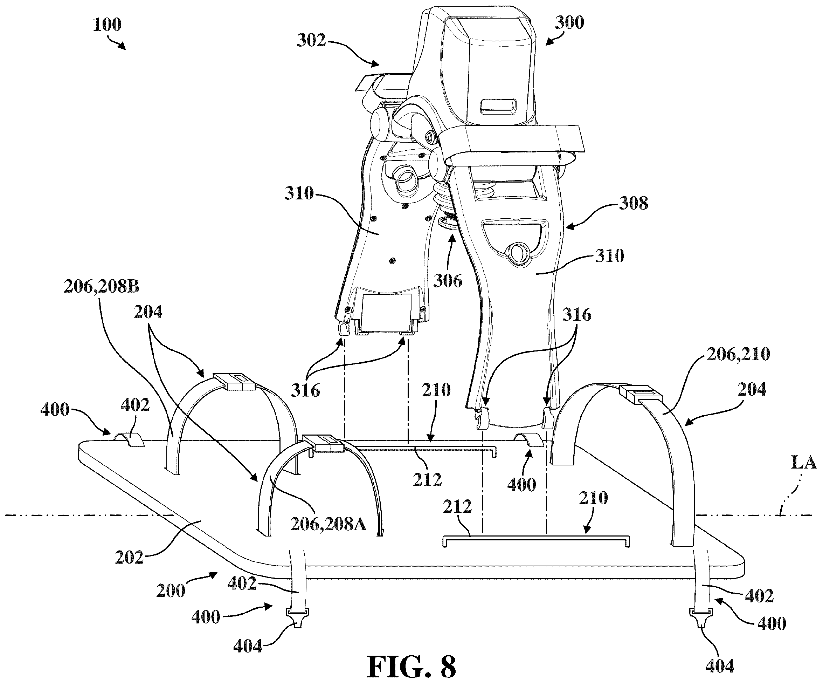

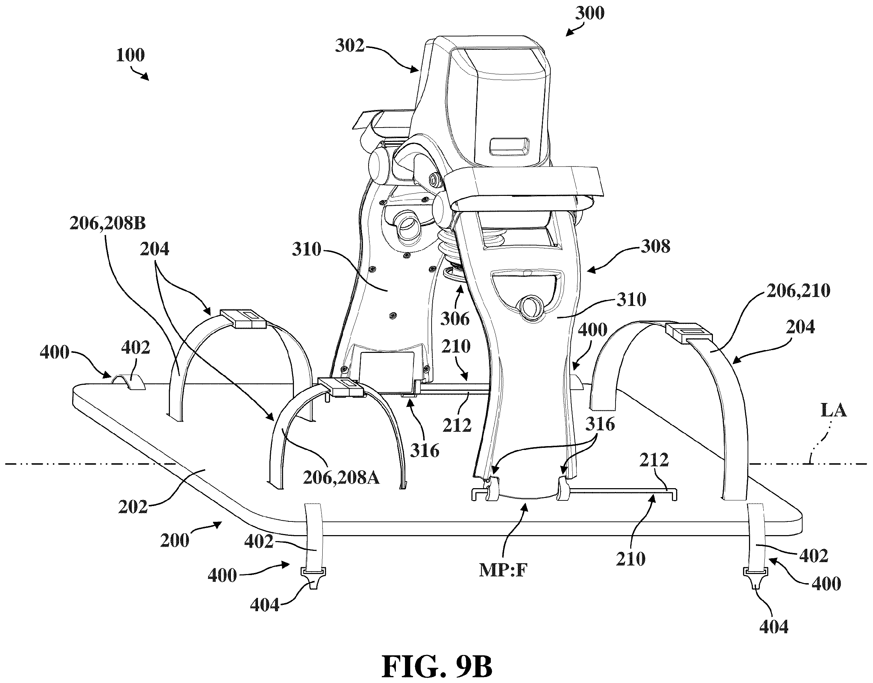

In another example, referring to Figures 8-9B, the backboard 202 may include a translator assembly 216 configured to be releasably coupled with the lateral driver mounts 310 and adjustable between the plurality of frame mounting positions MP. In the illustrated configuration, the backboard 202 includes two translator assemblies 216 coupled to the backboard on opposing sides of the longitudinal Axis LA. The translator assembly 216 may include a rail 218 or similar feature configured to engage the locking mechanisms 316 to releasably couple the lateral driver mounts 310 to the backboard 202. In this configuration, instead of the plurality of frame mounting positions MP being defined as a discrete plurality of frame mounting positions MP, the plurality of frame mounting positions MP is not limited to discrete points. In other words, the translator assembly 216 may enable the locking mechanisms 316 to be releasably coupled the lateral driver mounts 310 to the backboard 202 at any position along the rail 218, providing adjustability for a caretaker to align the chest compression system 300 with the chest of the patient P. For Example, FIG. 9A shows the locking mechanisms 316 of chest compression system 300 engaging the translator assembly 216 of the backboard assembly 200 such that the chest compression system 300 is disposed at a rearward frame mounting position MP:R. FIG. 9B shows the locking mechanisms 316 of chest compression system 300 engaging the translator assembly 216 of the backboard assembly 200 such that the chest compression system 300 is disposed at a forward frame mounting position MP:F. Other configurations of the backboard 202 to define the plurality of frame mounting positions MP along the longitudinal axis LA are contemplated.

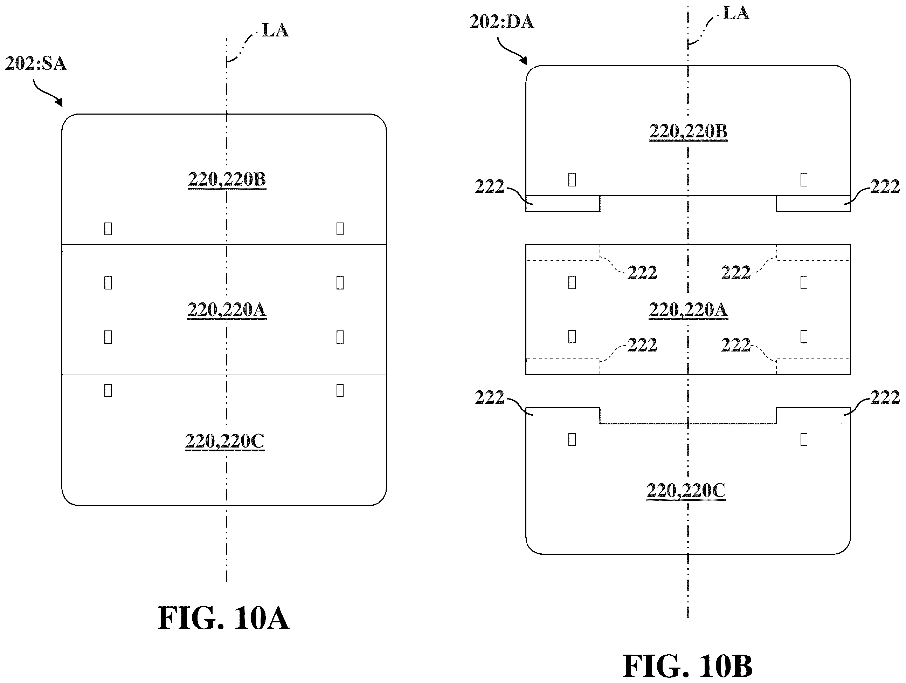

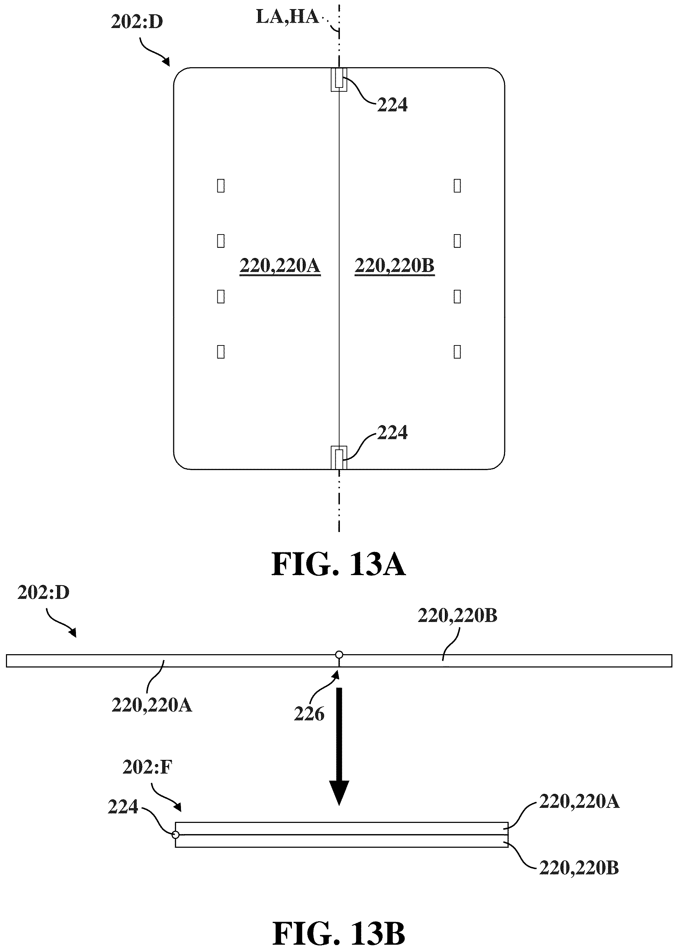

Referring to FIGS. 10A-14B, in some configurations, the backboard 202 includes a plurality of backboard members 220. In these configurations, the plurality of backboard members 220 are configured for selective movement relative to each other. Accordingly, as described in further detail below, in these configurations, the plurality of backboard members 220 may be disassembled and/or folded relative to each other to facilitate storage of the backboard 202 when not in use. In some configurations, such as shown in FIGS. 10A-12B, the backboard 202 includes three backboard members 220A, 220B, 220C. In other configurations, such as shown in FIGS. 13A-14B, the backboard 202 includes may include two backboard members 220A, 220B. In some configurations, the plurality of backboard members 220 may be substantially the same size, or, in other configurations, some of the plurality of backboard members 220 may be larger (e.g., wider and/or longer) than other backboard members 220. In some configurations, the backboard 202 may be substantially flat (such as shown in FIGS. 10A-14B), but in other configurations the backboard 202 may be curved for patient comfort and/or the prevent the patient P from shifting laterally.

Referring first to FIGS. 10A and 10B, in some examples, the plurality of backboard members 220 are configured to be releasably coupled to each other to define the backboard 202 in an assembled state SA (shown in FIG. 10A), and the plurality of backboard members 220 are configured to be decoupled from each other in a disassembled state DS (shown in FIG. 10B) for storage. One having skill in the art will appreciate that, in addition to having a smaller profile in the disassembled state DS to facilitate storage, the reduced size of the backboard 202 in the disassembled state DS may also advantageously improve portability of the backboard 202 when not in use. The plurality of backboard members 220 may be configured to be decoupled from each other in the disassembled state DS in a direction parallel to the longitudinal axis LA, as shown in FIG. 10B, but other configurations (e.g., transverse to the longitudinal axis LA) are contemplated. With continued reference to FIGS. 10A and 10B, in these examples, the plurality of backboard members 220 may each include one or more interlocking features 222 configured to cooperate with the one or more interlocking features 222 of another one of the plurality of backboard members 220 to releasably couple the plurality of backboard members 220 to each other to define the backboard 202 in the assembled state SA. The one or more interlocking features 222 may include but are not limited to snap-fit type joints, toggle or spring latches configured to engage corresponding catches, detent mechanisms, cam locks, magnetic connections, etc. Other configurations are contemplated.

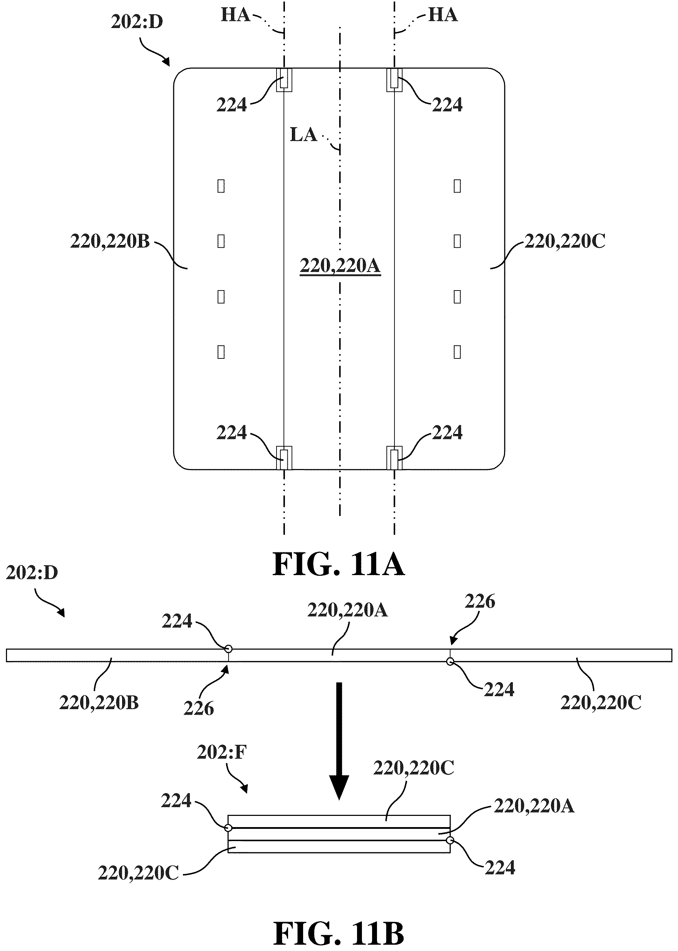

Referring to FIGS. 11A-14B, in other examples, the plurality of backboard members 220 may be hingedly connected to each other (i.e., connected by one or more hinges 224) such that the plurality of backboard members 220 is configured for movement between a folded position F (shown in FIGS. 11B, 12B, 13B, and 14B) where the plurality of backboard members 220 are folded relative to each other for storage, and a deployed position D (shown in FIGS. 11A, 12A, 13A, and 14A) where the plurality of backboard members 220 are unfolded relative to each other to define the backboard 202. In some examples, such as shown in FIGS. 11A-12B, the plurality of backboard members 220 may include a first backboard member 220A, a second backboard member 220B hingedly connected to the first backboard member 220A for movement between the folded position F and the deployed position D, and a third backboard member 220C hingedly connected to the first backboard member 220A for movement between the folded position F and the deployed position D. In other examples, such as shown in FIGS. 13A-14B, the plurality of backboard members 220 includes a first backboard member 220A and a second backboard member 220B hingedly connected to each other for movement between the folded position F and the deployed position D. In the illustrated configurations, the plurality of backboard members 220 have substantially the same size, but as described above, it should be appreciated that in other configurations, some of the plurality of backboard members 220 may be larger (e.g., wider and/or longer) than other backboard members 220.

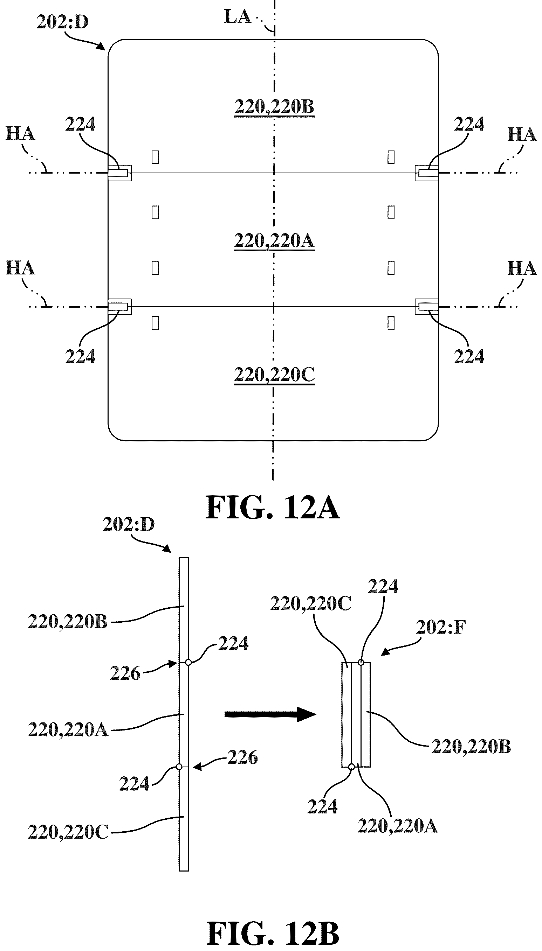

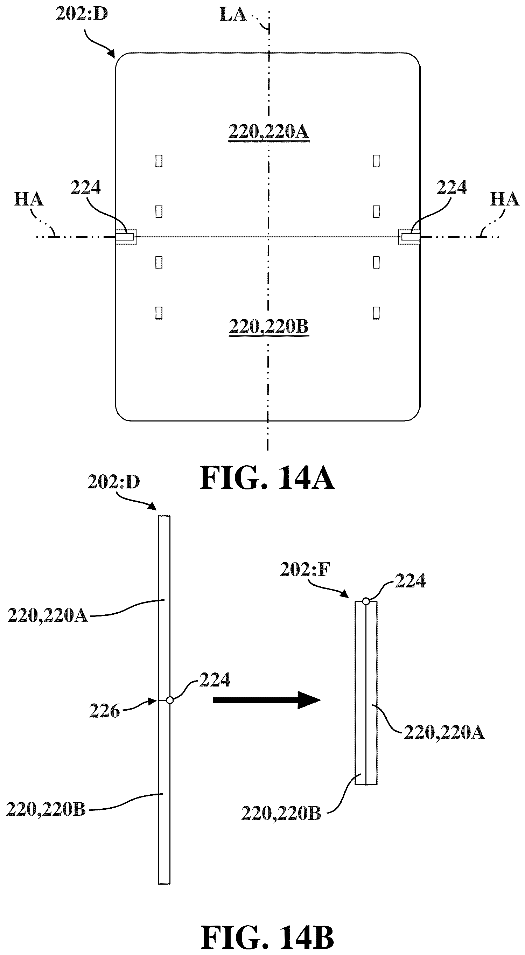

Referring to FIGS. 11A-11B and 13A-13B, in some examples, the plurality of backboard members 220 are configured to pivot about respective hinge axes HA parallel to the longitudinal axis LA. Referring to FIGS. 12A-12B and 14A-14B, in other examples, the plurality of backboard members 220 are configured to pivot about respective hinge axes HA transverse to the longitudinal axis LA. Additionally, as illustrated schematically in FIGS. FIGS. 11B, 12B, 13B, and 14B, the plurality of backboard members 220 may each include a stop portion 226 (e.g., a wall, a bumper, etc.) delimiting movement of the plurality of backboard members 220 relative to each other to define the deployed position D. Other configurations of disassembling and/or folding the plurality of backboard members 220 relative to each other to facilitate storage of the backboard 202 when not in use are contemplated.

Several configurations have been discussed in the foregoing description. However, the configurations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The present disclosure also comprises the following clauses, with specific features laid out in dependent clauses, that may specifically be implemented as described in greater detail with reference to the configurations and drawings above.

### CLAUSES

I. A patient care system for treating a patient, the patient care system comprising:
   a patient transport apparatus including:
      a base arranged for movement along floor surfaces,
      an intermediate frame arranged for movement relative to the base between a plurality of vertical configurations, and
      a patient support deck operatively attached to the intermediate frame and defining a patient support surface for supporting the patient;
   a backboard assembly configured to be coupled to the patient for concurrent movement with the patient, the backboard assembly including:
      a backboard defining a longitudinal axis, and
      a patient harness coupled to the backboard for coupling the backboard to the patient;
   a chest compression system configured to provide automatic chest compressions to the patient, the chest compression system including:
      a driver having a driver body movably supporting a plunger arranged for providing chest compressions to the patient, and
      a driver frame including lateral driver mounts configured to be releasably coupled to the backboard at a plurality of drive frame mounting positions along the longitudinal axis to support the driver adjacent to the chest of the patient; and
   a retainer for releasably coupling the backboard assembly to the intermediate frame of the patient transport apparatus in a coupled state to inhibit movement of the patient and the chest compression system relative to the patient support surface.
II. The patient care system according to clause I, wherein the retainer is operable between the coupled state and a decoupled state defined with the backboard assembly decoupled from the intermediate frame of the patient transport apparatus for permitting concurrent movement of the patient, the backboard assembly, and the chest compression system relative to the patient support surface such that the backboard assembly remains coupled to the patient for concurrent movement with the patient to allow the driver of the chest compression system to continue providing chest compressions to the patient as the retainer moves from the coupled state to the decoupled state.
III. The patient care system according to any one of clauses I or II, wherein the retainer includes a plurality of tethers extending between the backboard and the intermediate frame.
IV. The patient care system according to clause III, wherein the intermediate frame of the patient transport apparatus defines a plurality of mounting points configured to engage a respective tether of the plurality of tethers of the retainer to releasably couple the backboard assembly to the intermediate frame in the coupled state.
V. The patient care system according to clause IV, wherein the plurality of mounting points each includes a mounting strap coupled to the intermediate frame of the patient transport apparatus and a mounting ring supported by the mounting strap and configured to engage a respective tether of the plurality of tethers of the retainer to releasably couple the backboard assembly to the intermediate frame in the coupled state.
VI. The patient care system according to any one of clauses I to V, wherein the patient and the chest compression system are inhibited from moving relative to the patient support surface by virtue of the retainer coupling the backboard assembly to the intermediate frame of the patient transport apparatus in the coupled state such that the patient harness is not directly coupled to the intermediate frame in the coupled state.
VII. The patient care system according to any one of clauses I to VI, wherein the plurality of drive frame mounting positions is a discrete plurality of drive frame mounting positions, and the backboard defines a plurality of mounting features defining the discrete plurality of drive frame mounting positions and configured to be releasably coupled with the lateral driver mounts.
VIII. The patient care system according to any one of clauses I to VI, wherein the backboard includes a translator assembly configured to be releasably coupled with the lateral driver mounts and adjustable between the plurality of drive frame mounting positions.
IX. The patient care system according to any one of clauses I to VIII, wherein the backboard comprises a plurality of backboard members configured for selective relative movement relative to each other.
X. The patient care system according to clause IX, wherein the plurality of backboard members are configured to be releasably coupled to each other to define the backboard in an assembled state; and
   wherein the plurality of backboard members are configured to be decoupled from each other in a disassembled state for storage.
XI. The patient care system according to clause X, wherein the plurality of backboard members each include one or more interlocking features configured to cooperate with the one or more interlocking features of another one of the plurality of backboard members to releasably couple the plurality of backboard members to each other to define the backboard in the assembled state.
XII. The patient care system according to clause IX, wherein the plurality of backboard members are hingedly connected to each other; and
   wherein the plurality of backboard members are configured for movement between a folded position where the plurality of backboard members are folded relative to each other for storage, and a deployed position where the plurality of backboard members are unfolded relative to each other to define the backboard.
XIII. The patient care system according to clause XII, wherein the plurality of backboard members includes a first backboard member and a second backboard member hingedly connected to each other for movement between the folded position and the deployed position.
XIV. The patient care system according to clause XII, wherein the plurality of backboard members includes:
   a first backboard member;
   a second backboard member hingedly connected to the first backboard member for movement between the folded position and the deployed position; and
   a third backboard member hingedly connected to the first backboard member for movement between the folded position and the deployed position.
XV. The patient care system according to any one of clauses XII to XIV, wherein the plurality of backboard members each include a stop portion delimiting movement of the plurality of backboard members relative to each other to define the deployed position.
XVI. The patient care system according to any one of clauses XII to XV, wherein the plurality of backboard members are configured to pivot about respective hinge axes parallel to the longitudinal axis.
XVII. The patient care system according to any one of clauses XII to XV, wherein the plurality of backboard members are configured to pivot about respective hinge axes transverse to the longitudinal axis.
XVIII. The patient care system according to any one of clauses I to XVII, wherein the patient harness includes a plurality of straps configured to engage the patient to couple the backboard to the patient.
XIX. The patient care system according to clause XVIII, wherein the plurality of straps includes a pair of shoulder straps configured to engage the shoulders of the patient to couple the backboard to the patient.
XX. The patient care system according to clauses XVIII or XIX, wherein the plurality of straps includes torso straps configured to engage the torso of the patient to couple the backboard to the patient.
XXI. The patient care system according to any one of clauses I to XX, wherein the backboard defines a length along the longitudinal axis and defines a width along a lateral axis transverse to the longitudinal axis, wherein the length is greater than the width.
XXII. A patient care system for treating a patient, the patient care system comprising:
   a backboard assembly configured to be coupled to the patient for concurrent movement with the patient, the backboard assembly including:
      a backboard defining a longitudinal axis, the backboard including a plurality of backboard members configured for selective relative movement relative to each other, and
      a patient harness coupled to the backboard for coupling the backboard to the patient; and
   a chest compression system configured to provide automatic chest compressions to the patient, the chest compression system including:
      a driver having a driver body movably supporting a plunger arranged for providing chest compressions to the patient, and
      a driver frame including lateral driver mounts configured to be releasably coupled to the backboard at a plurality of drive frame mounting positions along the longitudinal axis to support the driver adjacent to the chest of the patient.
XXIII. The patient care system according to clause XXII, wherein the plurality of backboard members are configured to be releasably coupled to each other to define the backboard in an assembled state; and
   wherein the plurality of backboard members are configured to be decoupled from each other in a disassembled state for storage.
XXIV. The patient care system according to clause XXIII, wherein the plurality of backboard members each include one or more interlocking features configured to cooperate with the one or more interlocking features of another one of the plurality of backboard members to releasably couple the plurality of backboard members to each other to define the backboard in the assembled state.
XXV. The patient care system according to clause XXII, wherein the plurality of backboard members are hingedly connected to each other; and
   wherein the plurality of backboard members are configured for movement between a folded position where the plurality of backboard members are folded relative to each other for storage, and a deployed position where the plurality of backboard members are unfolded relative to each other to define the backboard.
XXVI. The patient care system according to clause XXV, wherein the plurality of backboard members includes a first backboard member and a second backboard member hingedly connected to each other for movement between the folded position and the deployed position.
XXVII. The patient care system according to clause XXV, wherein the plurality of backboard members includes:
   a first backboard member;
   a second backboard member hingedly connected to the first backboard member for movement between the folded position and the deployed position; and
   a third backboard member hingedly connected to the first backboard member for movement between the folded position and the deployed position.
XXVIII. The patient care system according to any one of clauses XXV to XXVII, wherein the plurality of backboard members each include a stop portion delimiting movement of the plurality of backboard members relative to each other to define the deployed position.
XXIX. The patient care system according to any one of clauses XXV to XXVIII, wherein the plurality of backboard members are configured to pivot about respective hinge axes parallel to the longitudinal axis.
XXX. The patient care system according to any one of clauses XXV to XXVIII, wherein the plurality of backboard members are configured to pivot about respective hinge axes transverse to the longitudinal axis.
XXXI. The patient care system according to any one of clauses XXII to XXX, wherein the patient harness includes a plurality of straps configured to engage the patient to couple the backboard to the patient.
XXXII. The patient care system according to clause XXXI, wherein the plurality of straps includes a pair of shoulder straps configured to engage the shoulders of the patient to couple the backboard to the patient.
XXXIII. The patient care system according to clauses XXXI or XXXII, wherein the plurality of straps includes torso straps configured to engage the torso of the patient to couple the backboard to the patient.
XXXIV. The patient care system according to any one of clauses XXII to XXXIII, wherein the backboard defines a length along the longitudinal axis and defines a width along a lateral axis transverse to the longitudinal axis, wherein the length is greater than the width.
XXXV. The patient care system according to any one of clauses XXII to XXXIV, wherein the plurality of drive frame mounting positions is a discrete plurality of drive frame mounting positions, and the backboard defines a plurality of mounting features defining the discrete plurality of drive frame mounting positions and configured to be releasably coupled with the lateral driver mounts.
XXXVI. The patient care system according to any one of clauses XXII to XXXIV, wherein the backboard includes a translator assembly configured to be releasably coupled with the lateral driver mounts and adjustable between the plurality of drive frame mounting positions. XXXVII. The patient care system according to any one of clauses XXII to XXXVI, further comprising a retainer for releasably coupling the backboard assembly to an intermediate frame of a patient transport apparatus in a coupled state to inhibit movement of the patient and the chest compression system relative to the patient transport apparatus.
XXXVIII. The patient care system according to according to clause XXXVII, wherein the retainer includes a plurality of tethers extending between the backboard and the intermediate frame.
XXXIX. A patient care system for treating a patient, the patient care system comprising:
   a patient transport apparatus including:
      a base arranged for movement along floor surfaces,
      an intermediate frame arranged for movement relative to the base between a plurality of vertical configurations, and
      a patient support deck operatively attached to the intermediate frame and defining a patient support surface for supporting the patient;
   a backboard assembly configured to be coupled to the patient for concurrent movement with the patient, the backboard assembly including:
      a backboard defining a longitudinal axis, and
      a patient harness coupled to the backboard for coupling the backboard to the patient;
   a chest compression system configured to provide automatic chest compressions to the patient, the chest compression system including:
      a driver having a driver body movably supporting a plunger arranged for providing chest compressions to the patient, and
      a driver frame including lateral driver mounts configured to be releasably coupled to the backboard at a plurality of frame mounting positions along the longitudinal axis to support the driver adjacent to the chest of the patient; and
   a retainer for releasably coupling the backboard assembly to the intermediate frame of the patient transport apparatus in a coupled state to inhibit movement of the patient and the chest compression system relative to the patient support surface.
XL. The patient care system according to clause XXXIX, wherein the retainer is operable between the coupled state and a decoupled state defined with the backboard assembly decoupled from the intermediate frame of the patient transport apparatus for permitting concurrent movement of the patient, the backboard assembly, and the chest compression system relative to the patient support surface such that the backboard assembly remains coupled to the patient for concurrent movement with the patient to allow the driver of the chest compression system to continue providing chest compressions to the patient as the retainer moves from the coupled state to the decoupled state. XLI. The patient care system according to clause XXXIX, wherein the retainer includes a plurality of tethers extending between the backboard and the intermediate frame.
XLII. The patient care system according to clause XLI, wherein the intermediate frame of the patient transport apparatus defines a plurality of mounting points configured to engage a respective tether of the plurality of tethers of the retainer to releasably couple the backboard assembly to the intermediate frame in the coupled state.
XLIII. The patient care system according to clause XLII, wherein the plurality of mounting points each includes a mounting strap coupled to the intermediate frame of the patient transport apparatus and a mounting ring supported by the mounting strap and configured to engage a respective tether of the plurality of tethers of the retainer to releasably couple the backboard assembly to the intermediate frame in the coupled state.
XLIV. The patient care system according to clause XXXIX, wherein the patient and the chest compression system are inhibited from moving relative to the patient support surface by virtue of the retainer coupling the backboard assembly to the intermediate frame of the patient transport apparatus in the coupled state such that the patient harness is not directly coupled to the intermediate frame in the coupled state.
XLV. The patient care system according to clause XXXIX, wherein the plurality of frame mounting positions is a discrete plurality of frame mounting positions, and the backboard defines a plurality of mounting features defining the discrete plurality of frame mounting positions and configured to be releasably coupled with the lateral driver mounts.
XLVI. The patient care system according to clause XXXIX, wherein the backboard includes a translator assembly configured to be releasably coupled with the lateral driver mounts and adjustable between the plurality of frame mounting positions.
XLVII. The patient care system according to clause XXXIX, wherein the backboard comprises a plurality of backboard members configured for selective relative movement relative to each other.
XLVIII. The patient care system according to clause XLVII, wherein the plurality of backboard members are configured to be releasably coupled to each other to define the backboard in an assembled state; and
   wherein the plurality of backboard members are configured to be decoupled from each other in a disassembled state for storage.
XLIX. The patient care system according to clause XLVIII, wherein the plurality of backboard members each include one or more interlocking features configured to cooperate with the one or more interlocking features of another one of the plurality of backboard members to releasably couple the plurality of backboard members to each other to define the backboard in the assembled state.
L. The patient care system according to clause XLVII, wherein the plurality of backboard members are hingedly connected to each other; and
   wherein the plurality of backboard members are configured for movement between a folded position where the plurality of backboard members are folded relative to each other for storage, and a deployed position where the plurality of backboard members are unfolded relative to each other to define the backboard.
LI. The patient care system according to clause L, wherein the plurality of backboard members includes a first backboard member and a second backboard member hingedly connected to each other for movement between the folded position and the deployed position.
LII. The patient care system according to clause L, wherein the plurality of backboard members includes:
   a first backboard member;
   a second backboard member hingedly connected to the first backboard member for movement between the folded position and the deployed position; and
   a third backboard member hingedly connected to the first backboard member for movement between the folded position and the deployed position.
LIII. The patient care system according to clause L, wherein the plurality of backboard members each include a stop portion delimiting movement of the plurality of backboard members relative to each other to define the deployed position.
LIV. The patient care system according to clause L, wherein the plurality of backboard members are configured to pivot about respective hinge axes parallel to the longitudinal axis.
LV. The patient care system according to clause L, wherein the plurality of backboard members are configured to pivot about respective hinge axes transverse to the longitudinal axis.
LVI. The patient care system according to clause XXXIX, wherein the patient harness includes a plurality of straps configured to engage the patient to couple the backboard to the patient.
LVII. The patient care system according to clause LVI, wherein the plurality of straps includes a pair of shoulder straps configured to engage the shoulders of the patient to couple the backboard to the patient.
LVIII. The patient care system according to clause LVI, wherein the plurality of straps includes torso straps configured to engage the torso of the patient to couple the backboard to the patient.
LIX. The patient care system according to clause XXXIX, wherein the backboard defines a length along the longitudinal axis and defines a width along a lateral axis transverse to the longitudinal axis, wherein the length is greater than the width.
LX. A patient care system for treating a patient, the patient care system comprising:
   a backboard assembly configured to be coupled to the patient for concurrent movement with the patient, the backboard assembly including:
      a backboard defining a longitudinal axis, the backboard including a plurality of backboard members configured for selective relative movement relative to each other, and
      a patient harness coupled to the backboard for coupling the backboard to the patient; and
   a chest compression system configured to provide automatic chest compressions to the patient, the chest compression system including:
      a driver having a driver body movably supporting a plunger arranged for providing chest compressions to the patient, and
      a driver frame including lateral driver mounts configured to be releasably coupled to the backboard at a plurality of frame mounting positions along the longitudinal axis to support the driver adjacent to the chest of the patient.
LXI. The patient care system according to clause LX, wherein the plurality of backboard members are configured to be releasably coupled to each other to define the backboard in an assembled state; and
   wherein the plurality of backboard members are configured to be decoupled from each other in a disassembled state for storage.
LXII. The patient care system according to clause LXI, wherein the plurality of backboard members each include one or more interlocking features configured to cooperate with the one or more interlocking features of another one of the plurality of backboard members to releasably couple the plurality of backboard members to each other to define the backboard in the assembled state.
LXIII. The patient care system according to clause LX, wherein the plurality of backboard members are hingedly connected to each other; and
   wherein the plurality of backboard members are configured for movement between a folded position where the plurality of backboard members are folded relative to each other for storage, and a deployed position where the plurality of backboard members are unfolded relative to each other to define the backboard.
LXIV. The patient care system according to clause LXIII, wherein the plurality of backboard members includes a first backboard member and a second backboard member hingedly connected to each other for movement between the folded position and the deployed position.
LXV. The patient care system according to clause LXIII, wherein the plurality of backboard members includes:
   a first backboard member;
   a second backboard member hingedly connected to the first backboard member for movement between the folded position and the deployed position; and
   a third backboard member hingedly connected to the first backboard member for movement between the folded position and the deployed position.
LXVI. The patient care system according to clause LXIII, wherein the plurality of backboard members each include a stop portion delimiting movement of the plurality of backboard members relative to each other to define the deployed position.
LXVII. The patient care system according to clause LXIII, wherein the plurality of backboard members are configured to pivot about respective hinge axes parallel to the longitudinal axis.
LXVIII. The patient care system according to clause LXIII, wherein the plurality of backboard members are configured to pivot about respective hinge axes transverse to the longitudinal axis.
LXIX. The patient care system according to clause LX, wherein the patient harness includes a plurality of straps configured to engage the patient to couple the backboard to the patient.
LXX. The patient care system according to clause LXIX, wherein the plurality of straps includes a pair of shoulder straps configured to engage the shoulders of the patient to couple the backboard to the patient.
LXXI. The patient care system according to clause LXIX, wherein the plurality of straps includes torso straps configured to engage the torso of the patient to couple the backboard to the patient.
LXXII. The patient care system according to clause LX, wherein the backboard defines a length along the longitudinal axis and defines a width along a lateral axis transverse to the longitudinal axis, wherein the length is greater than the width.
LXXIII. The patient care system according to clause LX, wherein the plurality of frame mounting positions is a discrete plurality of frame mounting positions, and the backboard defines a plurality of mounting features defining the discrete plurality of frame mounting positions and configured to be releasably coupled with the lateral driver mounts.
LXXIV. The patient care system according to clause LX, wherein the backboard includes a translator assembly configured to be releasably coupled with the lateral driver mounts and adjustable between the plurality of frame mounting positions.
LXXV. The patient care system according to clause LX, further comprising a retainer for releasably coupling the backboard assembly to an intermediate frame of a patient transport apparatus in a coupled state to inhibit movement of the patient and the chest compression system relative to the patient transport apparatus.
LXXVI. The patient care system according to according to clause LXXV, wherein the retainer includes a plurality of tethers extending between the backboard and the intermediate frame.

## Claims

1. A patient care system for treating a patient, the patient care system comprising:
a patient transport apparatus including:
a base arranged for movement along floor surfaces,
an intermediate frame arranged for movement relative to the base between a plurality of vertical configurations, and
a patient support deck operatively attached to the intermediate frame and defining a patient support surface for supporting the patient;
a backboard assembly configured to be coupled to the patient for concurrent movement with the patient, the backboard assembly including:
a backboard defining a longitudinal axis, and
a patient harness coupled to the backboard for coupling the backboard to the patient;
a chest compression system configured to provide automatic chest compressions to the patient, the chest compression system including:
a driver having a driver body movably supporting a plunger arranged for providing chest compressions to the patient, and
a driver frame including lateral driver mounts configured to be releasably coupled to the backboard at a plurality of drive frame mounting positions along the longitudinal axis to support the driver adjacent to the chest of the patient; and
a retainer for releasably coupling the backboard assembly to the intermediate frame of the patient transport apparatus in a coupled state to inhibit movement of the patient and the chest compression system relative to the patient support surface.

2. The patient care system according to claim 1, wherein the retainer is operable between the coupled state and a decoupled state defined with the backboard assembly decoupled from the intermediate frame of the patient transport apparatus for permitting concurrent movement of the patient, the backboard assembly, and the chest compression system relative to the patient support surface such that the backboard assembly remains coupled to the patient for concurrent movement with the patient to allow the driver of the chest compression system to continue providing chest compressions to the patient as the retainer moves from the coupled state to the decoupled state.

3. The patient care system according to any one of claims 1 or 2, wherein the retainer includes a plurality of tethers extending between the backboard and the intermediate frame.

4. The patient care system according to claim 3, wherein the intermediate frame of the patient transport apparatus defines a plurality of mounting points configured to engage a respective tether of the plurality of tethers of the retainer to releasably couple the backboard assembly to the intermediate frame in the coupled state.

5. The patient care system according to claim 4, wherein the plurality of mounting points each includes a mounting strap coupled to the intermediate frame of the patient transport apparatus and a mounting ring supported by the mounting strap and configured to engage a respective tether of the plurality of tethers of the retainer to releasably couple the backboard assembly to the intermediate frame in the coupled state.

6. The patient care system according to any one of claims 1 to 5, wherein the patient and the chest compression system are inhibited from moving relative to the patient support surface by virtue of the retainer coupling the backboard assembly to the intermediate frame of the patient transport apparatus in the coupled state such that the patient harness is not directly coupled to the intermediate frame in the coupled state.

7. The patient care system according to any one of claims 1 to 6, wherein the plurality of drive frame mounting positions is a discrete plurality of drive frame mounting positions, and the backboard defines a plurality of mounting features defining the discrete plurality of drive frame mounting positions and configured to be releasably coupled with the lateral driver mounts.

8. The patient care system according to any one of claims 1 to 6, wherein the backboard includes a translator assembly configured to be releasably coupled with the lateral driver mounts and adjustable between the plurality of drive frame mounting positions.

9. The patient care system according to any one of claims 1 to 8, wherein the backboard comprises a plurality of backboard members configured for selective relative movement relative to each other.

10. The patient care system according to claim 9, wherein the plurality of backboard members are configured to be releasably coupled to each other to define the backboard in an assembled state; and
wherein the plurality of backboard members are configured to be decoupled from each other in a disassembled state for storage.

11. The patient care system according to claim 10, wherein the plurality of backboard members each include one or more interlocking features configured to cooperate with the one or more interlocking features of another one of the plurality of backboard members to releasably couple the plurality of backboard members to each other to define the backboard in the assembled state.

12. The patient care system according to claim 9, wherein the plurality of backboard members are hingedly connected to each other; and
wherein the plurality of backboard members are configured for movement between a folded position where the plurality of backboard members are folded relative to each other for storage, and a deployed position where the plurality of backboard members are unfolded relative to each other to define the backboard.

13. The patient care system according to claim 12, wherein the plurality of backboard members includes a first backboard member and a second backboard member hingedly connected to each other for movement between the folded position and the deployed position.

14. The patient care system according to claim 12, wherein the plurality of backboard members includes:
a first backboard member;
a second backboard member hingedly connected to the first backboard member for movement between the folded position and the deployed position; and
a third backboard member hingedly connected to the first backboard member for movement between the folded position and the deployed position.

15. The patient care system according to any one of claims 12 to 14, wherein the plurality of backboard members each include a stop portion delimiting movement of the plurality of backboard members relative to each other to define the deployed position.
